# EUROPEAN PATENT APPLICATION

(11) **EP 3 023 783 A1**
(43) Date of publication of application: **25.05.2016**
(21) Application number: 14826219.9
(22) Date of filing: 16.07.2014
(51) Int. Cl.: G01N 31/00, G01N 21/77, G01N 21/78, G01N 31/22

(54) **OXYGEN DETECTION AGENT COMPOSITION, OXYGEN DETECTION SHEET, OXYGEN ABSORBER PACKAGING MATERIAL, AND OXYGEN ABSORBER PACKET**

(30) Priority: 16.07.2013 JP 2013147933
(71) Applicant: Mitsubishi Gas Chemical Company, Inc., Tokyo 100-8324 (JP)
(72) Inventor: NAKAMURA, Kazuhiro, Tokyo 125-8601 (JP); SUGITO, Ken, Tokyo 125-8601 (JP)
(74) Representative: Hoffmann Eitle
(86) International application number: PCT/JP2014/068913
(87) International publication number: WO 2015/008792

(57) **Abstract**

The provision of an oxygen detecting agent composition comprising polyethylene glycol, a redox dye, a reducing agent, and a basic substance, wherein a content of the polyethylene glycol is 5 to 35% by mass based on a total amount of the oxygen detecting agent composition, and the basic substance is a poorly water-soluble basic substance having a solubility in water of less than 1 g/100 g-H₂O at 20°C, the oxygen detecting agent composition comprising substantially no trisodium phosphate.

## Description

### Technical Field

The present invention relates to an oxygen detecting agent composition, an oxygen detecting sheet, a packaging material for an oxygen scavenger, and an oxygen scavenger package.

### Background Art

Conventionally, oxygen detecting agents utilizing organic dyes whose color changes reversibly by a redox reaction are used. Commercial oxygen detecting agents (for example, trade name "AGELESS EYE", manufactured by Mitsubishi Gas Chemical Company, Inc.) simply indicate a state in which oxygen is scavenged to the oxygen level of less than 0.1% by volume by its color change, and are used for retention of food freshness, retention of medical medicine quality, and the like together with oxygen scavengers (for example, trade name "AGELESS", manufactured by Mitsubishi Gas Chemical Company, Inc.).

Regarding oxygen detecting agent compositions, Patent Document 1 discloses an oxygen indicator label comprising ethylene glycol. Patent Document 2 discloses an oxygen indicator obtained by applying an agent having oxygen detecting ability comprising polyethylene glycol to a polyethylene powder sintered body. Patent Document 3 discloses a method for producing an oxygen detecting body obtained by impregnating with an oxygen detecting solution containing polyethylene glycol an absorber capable of being impregnated with a liquid. Patent Document 4 discloses an oxygen detecting agent composition containing polyethylene glycol.

### List of Prior Art Documents

### Patent Document

Patent Document 1: Japanese Patent Laid-Open No. 04-151555
Patent Document 2: Japanese Patent Laid-Open No. 02-057975
Patent Document 3: Japanese Patent Laid-Open No. 2007-298315
Patent Document 4: Japanese Patent Laid-Open No. 05-312799

### Summary of Invention

### Problems to be Solved by Invention

However, the present inventors have newly found the following problems regarding the above conventional art. The oxygen detecting agent composition described in Patent Document 1 gives a pink color at an oxygen concentration of less than 0.1% by volume, but may changes color from pink to yellow or brown when stored at ordinary temperature (for example, about 25 to 40°C) for a long period. In order to avoid this, it is necessary to control storage temperature at low temperature such as 15°C or less when storing the oxygen detecting agent composition for a long period. In addition, problems of the oxygen detecting agent compositions described in Patent Documents 2 to 4 are that depending on the average molecular weight of polyethylene glycol used in combination, the amount of the polyethylene glycol blended, the solubility of the basic substance in water, and the like, when the oxygen detecting agent compositions are stored at ordinary temperature for a long period, the color of the oxygen detecting agents changes to thereby fail to maintain the normal oxygen detecting function, and also the oxygen detecting agent compositions become sticky to thereby lack printability. Thus, problems of the conventional oxygen detecting agent compositions are that they have good responsiveness to oxygen concentration change and cannot maintain color retention properties and printability at high levels even when stored at ordinary temperature for a long period.

The present invention has been made in view of the above circumstances, and it is an object of the present invention to provide an oxygen detecting agent composition that has good responsiveness to oxygen concentration change and also has excellent color retention properties and printability even when stored at ordinary temperature for a long period.

### Means for Solving Problems

The present inventors have studied diligently in order to solve the above problems, and, as a result, unexpectedly found that in an oxygen detecting agent composition comprising polyethylene glycol, a redox dye, a reducing agent, and a basic substance, setting the content of the polyethylene glycol to a particular proportion and using a basic substance having low solubility in water provide an oxygen detecting agent composition that has good responsiveness to oxygen concentration change and also has excellent color retention properties and printability even when stored at ordinary temperature for a long period, thereby making the present invention.

Specifically, the present invention is as follows.
<1> An oxygen detecting agent composition comprising polyethylene glycol, a redox dye, a reducing agent, and a basic substance, wherein
   a content of the polyethylene glycol is 5 to 35% by mass based on a total amount of the oxygen detecting agent composition, and
   the basic substance is a poorly water-soluble basic substance having a solubility in water of less than 1 g/100 g-H₂O at 20°C,
   the oxygen detecting agent composition comprising substantially no trisodium phosphate.
<2> The oxygen detecting agent composition according to <1>, wherein an average molecular weight of the polyethylene glycol is 200 to 700.
<3> The oxygen detecting agent composition according to <1> or <2>, wherein the basic substance is an alkaline earth metal hydroxide, an alkaline earth metal carbonate, or both thereof.
<4> The oxygen detecting agent composition according to any one of <1> to <3>, wherein a content of a water-soluble basic substance having a solubility in water of 1 g/100 g-H₂O or more at 20°C in the oxygen detecting agent composition is 1% by mass or less.
<5> An oxygen detecting sheet comprising the oxygen detecting agent composition according to any one of <1> to <4>.
<6> A packaging material for an oxygen scavenger comprising the oxygen detecting sheet according to <5>.
<7> An oxygen scavenger package comprising:
   an oxygen scavenger composition; and
   the packaging material for the oxygen scavenger according to <6> packaging the oxygen scavenger composition.

### Advantages of Invention

According to the present invention, it is possible to provide an oxygen detecting agent composition that has good responsiveness to oxygen concentration change and also has excellent color retention properties and printability even when stored at ordinary temperature for a long period.

### Mode for Carrying Out Invention

A mode for carrying out the present invention (hereinafter simply referred to as "the present embodiment") will be described in detail below. The present embodiment below is an illustration for describing the present invention and is not intended to limit the present invention to the following contents. Appropriate modifications can be made to the present invention without departing from the spirit thereof.

An oxygen detecting agent composition of the present embodiment is an oxygen detecting agent composition comprising polyethylene glycol, a redox dye, a reducing agent, and a basic substance, wherein the content of the polyethylene glycol is 5 to 35% by mass based on the total amount of the oxygen detecting agent composition, and the basic substance is a poorly water-soluble basic substance having a solubility in water of less than 1 g/100 g-H₂O at 20°C, the oxygen detecting agent composition comprising substantially no trisodium phosphate. The oxygen detecting agent composition of the present embodiment has good responsiveness to oxygen concentration change and also has excellent color retention properties and printability even when stored at ordinary temperature for a long period. In addition, of course, the oxygen detecting agent composition of the present embodiment has good responsiveness to oxygen concentration change and also has excellent color retention properties and printability even when stored at low temperature. The ordinary temperature here is about 25 to 40°C. The components and the like will be described below.

### <Polyethylene Glycol>

The oxygen detecting agent composition of the present embodiment uses polyethylene glycol as a water retaining agent. The content of the polyethylene glycol in the oxygen detecting agent composition is 5 to 35% by mass. The content of the polyethylene glycol is preferably 10 to 30% by mass, more preferably 12 to 20% by mass. By setting the content of the polyethylene glycol in the above range, the redox reaction of the redox dye described later is promoted to thereby make the responsiveness to oxygen concentration change good, and also after storage at ordinary temperature for a long period, the color change of the oxygen detecting agent composition can be suppressed, while the stickiness of the oxygen detecting agent composition is suppressed to obtain good printability.

The average molecular weight of the polyethylene glycol is not particularly limited but is preferably 200 to 2000, more preferably 200 to 1000, further preferably 200 to 700, and still further preferably 300 to 600. The average molecular weight of the polyethylene glycol herein refers to average molecular weight obtained by the macrogol average molecular weight measurement method described in the Japanese Pharmacopoeia. Specifically, commercial "polyethylene glycol 400" (average molecular weight 400 ± about 15) and "polyethylene glycol 600" (average molecular weight 600 ± about 15) containing many fractions having a molecular weight of 400 to 600 can be used. One of these may be used alone, or two or more of these may be mixed in any proportion and used.

The content of a polyhydric alcohol other than polyethylene glycol is preferably 5% by mass or less, more preferably 0.5% by mass or less, further preferably 0.05% by mass or less, and still further preferably 0% by mass based on the total amount of the oxygen detecting agent composition. In other words, the oxygen detecting agent composition still further preferably does not comprise a polyhydric alcohol other than polyethylene glycol. In the oxygen detecting agent composition of the present embodiment, as the content of a polyhydric alcohol other than polyethylene glycol decreases, the color retention properties, color change performance, and the like of the oxygen detecting agent composition at ordinary temperature can be enhanced.

### <Redox Dye>

The redox dye in the present embodiment is not limited in any way as long as the color changes reversibly between an oxidized state and a reduced state. Examples of the redox dye include redox indicators such as ferroin and erioglaucine A; thiazine dyes such as methylene blue, new methylene blue, and methylene green; azine dyes such as safranine T and phenosafranine; oxazine dyes such as Nile blue; indigoid dyes such as indigo and indigo carmine; and thioindigoid dyes such as thioindigo. Among these, from the viewpoint of coloring power, atmosphere responsiveness, and durability, thiazine dyes are preferred. Among them, methylene blue is more preferred.

The content of the redox dye is not particularly limited as long as a change in color between an oxidized state and a reduced state can be visually observed. From the viewpoint of the color and color change speed of the composition, the content of the redox dye is preferably 0.01 to 5% by mass, more preferably 0.05 to 3% by mass, and further preferably 0.1 to 1% by mass based on the total amount of the oxygen detecting agent composition.

### <Reducing Agent>

The reducing agent used in the present embodiment should be a compound that can reduce the redox dye in an oxidized state even under conditions in which the oxygen concentration is lower than in the air. The type of the reducing agent is not particularly limited. As the reducing agent, known reducing agents can also be used. Specific examples of the reducing agent include monosaccharides such as glucose, fructose, and xylose, disaccharides such as maltose, ascorbic acid and salts thereof, dithionous acid and salts thereof, and cysteine and salts thereof. One of these may be used alone, or two or more of these may be used in combination. Among these, from the viewpoint of reducing power and safety, monosaccharides are preferred. Among them, fructose is more preferred, and D-fructose is further preferred.

The content of the reducing agent is not particularly limited but is preferably twice or more the amount of the redox dye on an amount-of-substance basis from the viewpoint of reduction reaction promotion. The molar ratio of the reducing agent to the redox dye (the reducing agent/the redox dye) is preferably 5 to 1000, more preferably 10 to 500, and further preferably 50 to 300.

### <Basic Substance>

The basic substance is a poorly water-soluble basic substance having a solubility in water of less than 1 g/100 g-H₂O at 20°C and is used in order to enhance the reduction activity of the reducing agent, and the like. Specific examples of the poorly water-soluble basic substance include, but are not limited to, hydroxides of alkaline earth metals such as calcium hydroxide and magnesium hydroxide; carbonates of alkaline earth metals such as magnesium carbonate and calcium carbonate; silicates such as calcium silicate, magnesium silicate, and aluminum silicate; and, among silica, zeolites, clay minerals, and the like, those that exhibit basicity when formed into water slurries. Among these, from the viewpoint of price and color change performance, alkaline earth metal hydroxides and alkaline earth metal carbonates are preferred, and alkaline earth metal hydroxides are more preferred. As long as the effect of the present embodiment is obtained, one basic substance may be used alone, or two or more basic substances may be used in combination. The oxygen detecting agent composition of the present embodiment preferably does not comprise a metal oxide such as aluminum oxide, zinc oxide, or magnesium oxide.

The oxygen detecting agent composition of the present embodiment does not substantially comprise trisodium phosphate (also including, for example, hydrates such as trisodium phosphate dodecahydrate) as a basic substance. Conventionally, some attempts have been made to blend a basic substance such as trisodium phosphate. But, as a result of diligent studies, the present inventors have unexpectedly found that the oxygen detecting agent composition of the present embodiment comprising substantially no trisodium phosphate has good responsiveness to oxygen concentration change and also has excellent color retention properties and printability even when stored at ordinary temperature for a long period. "Comprising substantially no trisodium phosphate" here means that the content of trisodium phosphate in the oxygen detecting agent composition is 0.8% by mass or less. The content of trisodium phosphate is preferably 0.5% by mass or less, more preferably 0.09% by mass or less, further preferably 0.01% by mass or less, and still further preferably 0% by mass. In other words, the oxygen detecting agent composition of the present embodiment further preferably comprises no trisodium phosphate.

The content of the poorly water-soluble basic substance in the oxygen detecting agent composition is preferably 20 to 70% by mass, more preferably 30 to 50% by mass. By setting the content of the poorly water-soluble basic substance in the above range, the responsiveness to oxygen concentration change is further quickened, and the color retention properties can be further enhanced.

In the oxygen detecting agent composition of the present embodiment, as long as its effect is not impaired, a water-soluble basic substance having a solubility in water of 1 g/100 g-H₂O or more at 20°C may be used in combination with the poorly water-soluble basic substance as a basic substance. The content of the water-soluble basic substance in the oxygen detecting agent composition is preferably 1% by mass or less, more preferably 0.1% by mass or less, further preferably 0.01% by mass or less, and still further preferably 0% by mass. In other words, the oxygen detecting agent composition still further preferably comprises no water-soluble basic substance. In the oxygen detecting agent composition of the present embodiment, as the content of the water-soluble basic substance decreases, the color retention properties of the oxygen detecting agent composition at ordinary temperature can be enhanced.

### <Binder>

A binder can be added to the oxygen detecting agent composition of the present embodiment as required. Examples of the binder include water-soluble polymers such as sodium alginate, gum arabic, gum tragacanth, carboxymethyl cellulose, hydroxyethyl cellulose, methyl cellulose, dextrin, polyvinyl alcohol, sodium polyacrylate, and polyacrylamide; celluloses such as ethyl cellulose, ethylhydroxyethyl cellulose, and cellulose acetyl propionate; and water-insoluble polymers such as vinyl acetate resins, butyral resins, polyester resins, acrylic resins, polyether resins, polyamide resins, and petroleum-based resins.

When the binder is used, a solvent may be used as required. For example, when a water-soluble polymer is used as the binder, water is preferred for the solvent. In addition, when a water-insoluble polymer is used as the binder, alcohols such as methanol, ethanol, and isopropyl alcohol; esters such as ethyl acetate and butyl acetate; ketones such as methyl ethyl ketone and methyl isobutyl ketone; and the like are preferred for the solvent. Among these, alcohols, esters, or mixed solvents obtained by mixing both at any ratio are more preferred. The content of the binder in the oxygen detecting agent composition of the present embodiment is preferably 1 to 90% by mass, more preferably 3 to 80% by mass, and further preferably 5 to 50% by mass.

When the oxygen detecting agent composition of the present embodiment comprises a binder and a solvent, its viscosity is preferably appropriately adjusted depending on how the oxygen detecting agent composition is used on a base material. For example, when a method of printing the oxygen detecting agent composition on a base material surface is performed as described later, adjustment is preferably performed so that suitable viscosity for the printing method is obtained because preferred viscosity is different depending on the printing method. The viscosity can be appropriately adjusted from the viewpoint of, for example, the strength of the coating film, film thinning, and stickiness suppression. For example, when the oxygen detecting agent composition is printed on a base material by gravure printing, the viscosity of the oxygen detecting agent composition is preferably 0.05 to 0.5 Pa·s, more preferably 0.1 to 0.3 Pa·s. When the oxygen detecting agent composition is printed on a base material by offset printing, the viscosity of the oxygen detecting agent composition is preferably 0.01 to 0.3 Pa·s, more preferably 0.05 to 0.1 Pa·s. The viscosity here can be measured by a B-type viscometer.

### <Colorant>

By further adding to the oxygen detecting agent composition of the present embodiment a colorant that does not change color depending on the oxygen concentration, the color change of the redox dye can be made more clear. Examples of the colorant include, but are not limited to, Red No. 104 and Red No. 106. For example, when oxygen detection in a container in which food or medicine is stored is performed, a food dye is preferably used as the colorant. The content of the colorant in the oxygen detecting agent composition is not particularly limited as long as the redox dye becoming colorless can be visually observed. From the viewpoint of the dispersibility or solubility of the dye in the composition, the content of the colorant is preferably 0.01 to 5% by mass, more preferably 0.05 to 3% by mass, and further preferably 0.1 to 1% by mass.

### <Other Additives>

Other additives other than the above can also be added to the oxygen detecting agent composition of the present embodiment as required. Examples of such additives include inorganic powders (for example, silica and zeolites) intended for the suppression of coating film stickiness due to the exudation of the liquid components in the composition, surface modifiers (for example, polydimethylsiloxane, long chain alkanes, and inorganic powders whose surfaces are hydrophobized) intended for the suppression of the blocking of the composition, and thickening agents intended for the prevention of the aggregation and sedimentation of the particles in the composition due to viscosity adjustment, and homogenization.

### <Method for Producing Oxygen Detecting Agent Composition>

The method for producing the oxygen detecting agent composition is not particularly limited, and the oxygen detecting agent composition can also be produced by mixing the above-described substances by a known method. For example, by impregnating a fibrous base material such as paper, a thread, or a nonwoven fabric with the oxygen detecting agent composition obtained by mixing the above redox dye, reducing agent, polyethylene glycol, basic substance, and solvent, and then drying the oxygen detecting agent composition, a thread-like or sheet-like (sometimes referred to as "film-like") oxygen detecting body can be obtained.

### <Oxygen Detecting Sheet, Packaging Material for Oxygen Scavenger, and Oxygen Scavenger Package>

The oxygen detecting agent composition of the present embodiment can be used as an oxygen detecting sheet, a packaging material for an oxygen scavenger, an oxygen scavenger package, and the like.

Examples of the oxygen detecting sheet (sometimes referred to as a "film") include an oxygen detecting sheet comprising the oxygen detecting agent composition of the present embodiment. Regarding a method for producing an oxygen detecting sheet, for example, an oxygen detecting sheet can be produced by providing a solution of a binder dissolved in a mixed solvent of an alcohol and an ester; adding polyethylene glycol, a redox dye, and a reducing agent to the solution; dispersing and mixing them to prepare an ink-like oxygen detecting agent composition; applying or impregnating the ink-like oxygen detecting agent composition to or into a base material; and then drying it.

The oxygen detecting agent composition can also be directly applied or printed on a base material, but for the purpose of the improvement of the adhesiveness of the composition to a base material, applying a filler between both may be effective. As the filler, varnishes of flexible resins such as polyurethane resins and natural rubbers can be used. In addition, further applying an over-primer on an applied layer of oxygen detecting agent composition (hereinafter sometimes referred to as an "oxygen detecting agent layer", a "layer comprising the oxygen detecting agent composition", or the like) may be effective for the protection of the oxygen detecting agent layer. As the over-primer, the same ones as the above filler can be used.

Examples of the base material include plastic base materials such as polyesters, polyolefins, and polyvinyl chloride and fibrous base materials such as paper and nonwoven fabrics. When the oxygen detecting agent composition is liquid, a method such as applying, impregnating, or printing the oxygen detecting agent composition with respect to the base material can be adopted. Examples of the method for applying the oxygen detecting agent composition to the base material include methods using a spray, a bar coater, or the like. Examples of the method for printing the oxygen detecting agent composition on the base material include an offset printing method, a gravure printing method, a screen printing method, a flexographic printing method, and a letterpress printing method.

The base material may be a single layer or a plurality of layers. In the case of a configuration having a plurality of layers in which an oxygen detecting agent layer is sandwiched between other layers, it is necessary to make at least either the layer above or below the applied layer substantially transparent in order to make the color of the applied portion visible.

The drying temperature when the above-described liquid oxygen detecting agent composition is applied to the base material is not particularly limited as long as the solvent and the like can be volatilized. From the viewpoint of preventing the color change of the oxygen detecting agent composition due to heat, the drying temperature is preferably 100°C or less, more preferably 80°C or less, and further preferably 70°C or less.

In the present embodiment, a packaging material for an oxygen scavenger comprising the above-described oxygen detecting sheet can be provided. When the oxygen detecting sheet is used as a packaging material, a configuration having a plurality of layers such as an outer surface layer/an oxygen detecting agent layer (a layer comprising the oxygen detecting agent composition)/an inner surface layer is preferred. In other words, a configuration which has at least an outer surface layer, an oxygen detecting agent layer provided on this outer surface layer, and an inner surface layer provided on the oxygen detecting agent layer and in which the oxygen detecting agent layer is a layer comprising the oxygen detecting agent composition, and the like are preferred. Here, the inner surface layer is a layer in contact with an oxygen scavenger composition. The outer surface layer is a layer in contact with the outside and is a surface layer positioned opposite to the oxygen scavenger composition stored inside. Therefore, when an oxygen scavenger composition is packaged in the packaging material for an oxygen scavenger, for example, a configuration in which they are in contact in the order of the outer surface layer/the oxygen detecting agent layer/the inner surface layer/the oxygen scavenger composition can be adopted.

The inner surface layer directly packages the oxygen scavenger composition and therefore is preferably a layer having heat sealing properties (heat sealing layer). For example, the inner surface layer is preferably a layer comprising a low density polyethylene film or the like.

The outer surface layer is a layer in contact with the outside and therefore is preferably a layer comprising polyethylene terephthalate or the like.

From the viewpoint of avoiding the elution of various components contained in the oxygen detecting agent composition, it is preferred to further have a layer comprising a transparent resin on the oxygen detecting agent layer as a protective layer. The protective layer should be such that the color change of the oxygen detecting agent layer can be visually recognized from the outside. The protective layer may be provided between the outer surface layer and the oxygen detecting agent layer or between the inner surface layer and the oxygen detecting agent layer. As the method for providing the protective layer on the oxygen detecting agent layer, it is preferred to provide a portion (layer) comprising the oxygen detecting agent composition by the reverse printing of a film. As the lamination method after printing, dry lamination is preferred from the viewpoint that the oxygen detecting agent portion is less likely to be exposed to high temperature.

Examples of the oxygen scavenger package of the present embodiment include an oxygen scavenger package comprising an oxygen scavenger composition and the above-described packaging body for the oxygen scavenger packaging the oxygen scavenger composition. The oxygen scavenger composition should be one that can absorb oxygen and form an oxygen-scavenged state (for example, oxygen concentration 0.1% or less), and is not particularly limited.

The method for packaging the oxygen scavenger composition in the packaging body for the oxygen scavenger is not particularly limited, and known packaging machines and packaging methods, for example, three-side seal packaging by a rotary packer or the like, pillow packaging by a stick packer or the like, and four-side seal packaging by a four-side seal packaging machine or the like, can be used. Among these, three-side seal packaging by a rotary packer or the like is preferred from the viewpoint of production efficiency.

### Examples

The present invention will be more specifically described below by giving Examples and Comparative Examples. The present invention is not limited to these Examples.

In the following Examples, for isopropyl alcohol (hereinafter described as "IPA"), ethyl acetate, methylene blue, methylene green, D-fructose, D-xylose, magnesium hydroxide, magnesium carbonate, trisodium phosphate dodecahydrate, glycerin, polyethylene glycol 200 (hereinafter described as "PEG200"), and all water retaining agents shown in Table 1, reagents manufactured by Wako Pure Chemical Industries, Ltd. were used unless otherwise noted.

### (Example 1)

### <Making of Oxygen Indicator Ink>

A mixed solvent of 4.5 g of IPA and 4.5 g of ethyl acetate was provided, and 1.0 g of cellulose acetate propionate (trade name "504-0.2", manufactured by EASTMAN CHEMICAL, hereinafter described as "CAP") as a binder was dissolved. 0.016 g of methylene blue as a redox dye, 0.011 g of phloxine B (Food Red No. 104, manufactured by Hodogaya Chemical Co., Ltd.) as a colorant, 3.6 g of PEG200 as a water retaining agent (the content of PEG200 based on the total amount of the oxygen detecting agent composition after drying was 25% by mass), and 2.1 g of D-fructose as a reducing agent were added to this solution and dispersed to obtain an ink A.

In addition, 1.4 g of CAP was dissolved in a mixed solvent of 5.8 g of IPA and 5.8 g of ethyl acetate. 10 g of magnesium hydroxide (solubility in water at 20°C: 0.9 mg/100 g-H₂O) as a poorly water-soluble basic substance was mixed and dispersed in this solution to obtain an ink B.

1.1 g of the ink A, 1.1 g of the ink B, 0.7 g of IPA, and 0.7 g of ethyl acetate were mixed to obtain an oxygen indicator ink.

### <Making of Oxygen Detecting Sheet>

The ink was applied by the following procedure to a surface of YUPO Paper (FPD-80, manufactured by Yupo Corporation) cut to 100 mm x 150 mm. The application of the ink was performed using a bar coater (manufactured by TESTER SANGYO CO. LTD.). First, "CLIOS Medium (A)" (manufactured by DIC Graphics Corporation) as a protective layer was applied and dried by warm air at 60°C for 10 seconds. Then, the oxygen indicator ink obtained as an oxygen detecting layer was applied and dried by warm air at 60°C for 10 seconds. Finally, once again "CLIOS Medium" (A) (manufactured by DIC Graphics Corporation) was applied and dried by warm air at 60°C for 10 seconds to obtain an oxygen detecting sheet.

### <Color Change Performance Evaluation>

The obtained oxygen detecting sheet was cut to a size of 5 mm x 15 mm and fixed in an oxygen barrier bag with a tape. Then, humidity control cotton for controlling humidity to 80% RH, an oxygen scavenger (manufactured by Mitsubishi Gas Chemical Company, Inc., "AGELESS SA-100"; self-reactive iron-based oxygen scavenger), and 500 mL of nitrogen were enclosed and sealed in the bag. After the oxygen concentration in the bag reaching less than 0.1% by volume was checked using gas chromatography (manufactured by SHIMADZU CORPORATION, "GC-14A"), the bag was stored at 25°C, and the color tone of the oxygen detecting sheet was visually observed every 6 hours. A case where the time required by the color changing to pink, which indicated that the oxygen concentration reached less than 0.1% by volume, was within 6 hours was evaluated as "A", a case where the time was within 12 hours was evaluated as "B", and a case where the time was 18 hours or more was evaluated as "C". The humidity control cotton for controlling humidity to 80% RH was made by mixing 2.55 g of glycerin and 2.45 g of water and impregnating absorbent cotton with the mixture.

### <Color Retention Properties Evaluation>

The obtained oxygen detecting sheet was cut to a size of 5 mm x 15 mm and fixed in an oxygen barrier bag with a tape. Then, an oxygen scavenger (manufactured by Mitsubishi Gas Chemical Company, Inc., "AGELESS SA-202", self-reactive iron-based oxygen scavenger), humidity control cotton for controlling humidity to 70% RH, and 250 mL of air were enclosed and sealed in the bag. After the sealed bag was stored at 35°C for 1 month, the color of the oxygen detecting sheet in the sealed bag was visually observed. A case where there was no change in color before and after the storage was evaluated as "A", a case where the color changed but the oxygen concentration was visually recognized was evaluated as "B", and a case where the color changed and it was difficult to visually recognize the oxygen concentration was evaluated as "C". The result is shown in Table 1. The humidity control cotton for controlling humidity to 70% RH was made by mixing 3.15 g of glycerin and 1.85 g of water and impregnating absorbent cotton with the mixture.

### <Printability Evaluation>

Two of the obtained oxygen detecting sheets were provided, and one oxygen detecting sheet (hereinafter described as an "oxygen detecting sheet A") was placed so that the ink-applied surface faced upward. Next, the other oxygen detecting sheet (hereinafter described as an "oxygen detecting sheet B") was placed on top of the oxygen detecting sheet A so that the ink-applied surface faced upward. Then, the state of ink adhesion on the surface of the oxygen detecting sheet B to which the ink was not applied (the surface in contact with the ink-applied surface of the oxygen detecting sheet A) was visually observed. A surface without ink adhesion was evaluated as "A", and a surface with ink adhesion was evaluated as "C". The result is shown in Table 1.

### (Examples 2 to 6 and Comparative Examples 1 to 8)

An oxygen detecting agent and an oxygen detecting sheet were made as in Example 1 except that instead of PEG200, a substance shown in Table 1 was used as the water retaining agent. Then, for these, color change performance evaluation, color retention properties evaluation, and printability evaluation were performed as in Example 1. The results are shown in Table 1. Regarding the average molecular weight described in Table 1, the average molecular weight of polyethylene glycol was obtained by the macrogol average molecular weight measurement method described in the Japanese Pharmacopoeia.

The polyethylene glycol used is shown below.
"Polyethylene glycol 200" (average molecular weight: 190 to 210)
"Polyethylene glycol 300" (average molecular weight: 280 to 320)
"Polyethylene glycol 400" (average molecular weight: 380 to 420)
"Polyethylene glycol 600" (average molecular weight: 570 to 630)
"Polyethylene glycol 1000" (average molecular weight: 900 to 1100)
"Polyethylene glycol 1540" (average molecular weight: 1500)

The substances used other than polyethylene glycol are shown below.
Ethylene glycol (manufactured by Wako Pure Chemical Industries, Ltd., molecular weight 62)
Propylene glycol (manufactured by Wako Pure Chemical Industries, Ltd., molecular weight 76)
Dipropylene glycol (manufactured by Wako Pure Chemical Industries, Ltd., molecular weight 134)
Propylene glycol monomethyl ether (manufactured by Wako Pure Chemical Industries, Ltd., molecular weight 90)
Diethylene glycol (manufactured by Wako Pure Chemical Industries, Ltd., molecular weight 106)
Polypropylene glycol 300 (manufactured by Wako Pure Chemical Industries, Ltd., molecular weight described on the product: 300)
Polypropylene glycol 700 (manufactured by Wako Pure Chemical Industries, Ltd., molecular weight described on the product: 700)
Polypropylene glycol 2000 (manufactured by Wako Pure Chemical Industries, Ltd., molecular weight described on the product: 2000)

**[Table 1]**

| | Water retaining agent | Color change performance | Color retention properties | Printability |
|---|---|---|---|---|
| Example 1 | Polyethylene glycol 200 | A | A | A |
| Example 2 | Polyethylene glycol 300 | A | A | A |
| Example 3 | Polyethylene glycol 400 | A | A | A |
| Example 4 | Polyethylene glycol 600 | A | A | A |
| Example 5 | Polyethylene glycol 1000 | A | B | A |
| Example 6 | Polyethylene glycol 1540 | A | B | A |
| Comparative Example 1 | Ethylene glycol | A | C | A |
| Comparative Example 2 | Propylene glycol | A | C | A |
| Comparative Example 3 | Dipropylene glycol | A | C | A |
| Comparative Example 4 | Propylene glycol monomethyl ether | C | C | A |
| Comparative Example 5 | Diethylene glycol | A | B | A |
| Comparative Example 6 | Polypropylene glycol 300 | A | C | A |
| Comparative Example 7 | Polypropylene glycol 700 | A | C | A |
| Comparative Example 8 | Polypropylene glycol 2000 | A | C | A |

Redox dye: methylene blue
Reducing agent: D-fructose
Poorly water-soluble basic substance: magnesium hydroxide
Water retaining agent content: 25% by mass

In Examples 1 to 6 using polyethylene glycol as the water retaining agent, the color changed to pink within 6 hours after the start of the test, and the color retention properties and the printability were good. On the other hand, in Comparative Examples 1 to 8 using a water retaining agent other than polyethylene glycol, the result of at least either the color change performance or the color retention properties was insufficient.

### (Examples 7 to 12 and Comparative Examples 9 to 10)

An oxygen detecting agent and an oxygen detecting sheet were made as in Example 1 except that the amount of PEG200 blended was changed to a content (% by mass) shown in Table 2. In other words, the "PEG content" described in Table 2 represents the content (% by mass) of PEG200 based on the total amount of an oxygen detecting agent composition after drying. Then, for these, performance evaluation was performed as in Example 1. The results are shown in Table 2.

**[Table 2]**

| | Amount of PEG200 blended in ink A [g] | PEG200 content (% by mass) | Color change performance | Color retention properties | Printability |
|---|---|---|---|---|---|
| Example 7 | 0.9 | 9 | A | B | A |
| Example 8 | 1.8 | 15 | A | A | A |
| Example 9 | 2.7 | 20 | A | A | A |
| Example 10 | 3.6 | 25 | A | A | A |
| Example 11 | 4.5 | 28 | A | A | A |
| Example 12 | 5.4 | 31 | A | A | A |
| Comparative Example 9 | 0.2 | 2 | A | C | A |
| Comparative Example 10 | 9.0 | 40 | A | A | C |

According to Table 2, it was confirmed that particularly in Examples 8 to 12, all of the color change performance, the color retention properties, and the printability were better.

### (Example 13 and Comparative Example 11)

An oxygen detecting agent and an oxygen detecting sheet were made as in Example 1 except that instead of methylene blue, methylene green was used as the redox dye (Example 13). In addition, an oxygen detecting agent and an oxygen detecting sheet were made as in Comparative Example 1 except that instead of methylene blue, methylene green was used as the redox dye (Comparative Example 11). For each of Example 13 and Comparative Example 11, performance evaluation was performed. In Example 13, all of the color change performance, the color retention properties, and the printability were evaluated as "A", but in Comparative Example 11, the color retention properties were evaluated as "C".

### (Example 14 and Comparative Example 12)

An oxygen detecting agent and an oxygen detecting sheet were made as in Example 1 except that instead of D-fructose, D-xylose was used as the reducing agent (Example 14). In addition, an oxygen detecting agent and an oxygen detecting sheet were made as in Comparative Example 1 except that instead of D-fructose, D-xylose was used as the reducing agent (Comparative Example 12). For each of Example 14 and Comparative Example 12, performance evaluation was performed. In Example 14, all of the color change performance, the color retention properties, and the printability were evaluated as "A", but in Comparative Example 12, the color retention properties were evaluated as "C".

### (Example 15 and Comparative Example 13)

An oxygen detecting agent and an oxygen detecting sheet were made as in Example 1 except that instead of magnesium hydroxide, magnesium carbonate (solubility in water at 20°C: 0.039 g/100 g-H₂O) was used as the poorly water-soluble basic substance (Example 15). In addition, an oxygen detecting agent and an oxygen detecting sheet were made as in Comparative Example 1 except that instead of magnesium hydroxide, magnesium carbonate was used as the poorly water-soluble basic substance (Comparative Example 13). For each of Example 15 and Comparative Example 13, performance evaluation was performed. In Example 15, all of the color change performance, the color retention properties, and the printability were evaluated as "A", but in Comparative Example 13, the color retention properties were evaluated as "C".

### (Comparative Example 14)

An oxygen detecting agent and an oxygen detecting sheet were made and performance evaluation was performed as in Example 1 except that 0.4 g of trisodium phosphate dodecahydrate (solubility in water at 20°C: 12.1 g/100 g-H₂O (in terms of an anhydride)) (the content of trisodium phosphate dodecahydrate based on the total amount of the oxygen detecting agent composition after drying was 2% by mass, and the content of trisodium phosphate in terms of an anhydride was 0.86% by mass) was further added to the ink B and mixed and dispersed. In Comparative Example 14, the color retention properties were evaluated as "C".

The results of Examples 13 to 15 and Comparative Examples 11 to 14 are shown in Table 3.

**[Table 3]**

| | Redox dye | Reducing agent | Water retaining agent | Poorly water-soluble basic substance | Water-soluble basic substance | | Color change performance | Color retention properties | Printability |
|---|---|---|---|---|---|---|---|---|---|
| | | | | | Type | Amount blended in ink B [g] | | | |
| Example 13 | Methylene green | D-fructose | PEG200 | Magnesium hydroxide | None | - | A | A | A |
| Example 14 | Methylene blue | D-Xylose | PEG200 | Magnesium hydroxide | None | - | A | A | A |
| Example 15 | Methylene blue | D-fructose | PEG200 | Magnesium carbonate | None | - | A | A | A |
| Comparative Example 11 | Methylene green | D-fructose | Ethylene glycol | Magnesium hydroxide | None | - | A | C | A |
| Comparative Example 12 | Methylene blue | D-Xylose | Ethylene glycol | Magnesium hydroxide | None | - | A | C | A |
| Comparative Example 13 | Methylene blue | D-fructose | Ethylene glycol | Magnesium carbonate | None | - | A | C | A |
| Comparative Example 14 | Methylene blue | D-fructose | PEG200 | Magnesium hydroxide | Trisodium phosphate dodecahydrate | 0.4 | A | C | A |

This application is based on Japanese Patent Application No. 2013-147933 filed with the Japan Patent Office on July 16, 2013, the contents of which are incorporated herein by reference.

### Industrial Applicability

The oxygen detecting agent composition, the oxygen detecting sheet, the packaging material for an oxygen scavenger, and the oxygen scavenger package according to the present invention are capable of long-term storage and distribution at ordinary temperature. Thus, they need not be stored at low temperature, are easily managed, and have a large energy saving effect, and therefore they have applicability in a wide range of fields including the storage of food and medicine, the storage of metal products and rubber products, atmosphere control and management, and microbial culture and cell culture.

## Claims

1. An oxygen detecting agent composition comprising polyethylene glycol, a redox dye, a reducing agent, and a basic substance, wherein
a content of the polyethylene glycol is 5 to 35% by mass based on a total amount of the oxygen detecting agent composition, and
the basic substance is a poorly water-soluble basic substance having a solubility in water of less than 1 g/100 g-H₂O at 20°C,
the oxygen detecting agent composition comprising substantially no trisodium phosphate.

2. The oxygen detecting agent composition according to claim 1, wherein an average molecular weight of the polyethylene glycol is 200 to 700.

3. The oxygen detecting agent composition according to claim 1 or 2, wherein the basic substance is an alkaline earth metal hydroxide, an alkaline earth metal carbonate, or both thereof.

4. The oxygen detecting agent composition according to any one of claims 1 to 3, wherein a content of a water-soluble basic substance having a solubility in water of 1 g/100 g-H₂O or more at 20°C in the oxygen detecting agent composition is 1% by mass or less.

5. An oxygen detecting sheet comprising the oxygen detecting agent composition according to any one of claims 1 to 4.

6. A packaging material for an oxygen scavenger comprising the oxygen detecting sheet according to claim 5.

7. An oxygen scavenger package comprising:
an oxygen scavenger composition; and
the packaging material for the oxygen scavenger according to claim 6 packaging the oxygen scavenger composition.
